# EUROPÄISCHE PATENTSCHRIFT

(11) **EP 2 344 250 B1**
(45) Veröffentlichungstag und Bekanntmachung des Hinweises auf die Patenterteilung: **06.12.2017**
(21) Anmeldenummer: 09744065.5
(22) Anmeldetag: 17.10.2009
(51) Int. Cl.: A61N 5/10

(54) **BESTRAHLUNG EINES ZIELVOLUMENS UNTER BERÜCKSICHTIGUNG EINES ZU SCHÜTZENDEN VOLUMENS**
IRRADIATION OF A TARGET VOLUME, TAKING INTO ACCOUNT A VOLUME TO BE PROTECTED
IRRADIATION D'UN VOLUME CIBLE EN PRENANT EN CONSIDÉRATION UN VOLUME À PRÉSERVER

(30) Priorität: 27.10.2008 DE 102008053611
(43) Veröffentlichungstag der Anmeldung: 20.07.2011
(73) Patentinhaber: GSI Helmholtzzentrum für Schwerionenforschung GmbH, 64291 Darmstadt (DE); Siemens Aktiengesellschaft, 80333 München (DE)
(72) Erfinder: BERT, Christoph, 63741 Aschaffenburg (DE); RIETZEL, Eike, 64331 Weiterstadt (DE); KRAFT, Gerhard, 64291 Darmstadt (DE)
(74) Vertreter: Mergel, Volker
(86) Internationale Anmeldenummer: PCT/EP2009/007462
(87) Internationale Veröffentlichungsnummer: WO 2010/049072

(56) Entgegenhaltungen:
- EP-A1- 1 238 684
- EP-A1- 1 818 078
- WO-A2-03/076003
- US-A- 5 602 892
- US-A- 5 647 663
- US-A1- 2006 067 469
- US-A1- 2007 041 497
- US-A1- 2007 053 490
- US-A1- 2007 127 623
- MUREN L P ET AL: "Testing the new ICRU 62 'Planning Organ at Risk Volume' concept for the rectum" RADIOTHERAPY AND ONCOLOGY, ELSEVIER, Bd. 75, Nr. 3, 1. Juni 2005 (2005-06-01), Seiten 293-302, XP025290882 ISSN: 0167-8140 [gefunden am 2005-06-01]
- MCKENZIE ALAN ET AL: "Margins for geometric uncertainty around organs at risk in radiotherapy" RADIOTHERAPY AND ONCOLOGY, ELSEVIER, Bd. 62, Nr. 3, 1. März 2002 (2002-03-01), Seiten 299-307, XP007910937 ISSN: 0167-8140
- CEDRIC X YU ET AL: "CLINICAL IMPLEMENTATION OF INTENSITY-MODULATED ARC THERAPY", INTERNATIONAL JOURNAL OF RADIATION: ONCOLOGY BIOLOGY PHYSICS, PERGAMON PRESS, USA, vol. 53, no. 2, 1 January 2002 (2002-01-01), pages 453-463, XP007907772, ISSN: 0360-3016, DOI: 10.1016/S0360-3016(02)02777-3
- EUGENE WONG ET AL: "INTENSITY-MODULATED ARC THERAPY SIMPLIFIED", INTERNATIONAL JOURNAL OF RADIATION: ONCOLOGY BIOLOGY PHYSICS, PERGAMON PRESS, USA, vol. 53, no. 1, 1 January 2002 (2002-01-01), pages 222-235, XP007907774, ISSN: 0360-3016, DOI: 10.1016/S0360-3016(02)02735-9

## Beschreibung

Die Offenbarung betrifft ein Verfahren zur Planung einer Bestrahlung mit einem Zielpunkte anfahrenden Strahl, ein entsprechendes Verfahren zur Bestrahlung eines Phantoms , eine Vorrichtung für eine entsprechende Bestrahlung und ein Kontrollsystem zur Steuerung einer solchen Vorrichtung.

Die Bestrahlung eines Ziels mit einem verschiedene Punkte anfahrenden Strahl (Beam Scanning) ist an sich bekannt. So werden etwa bei der Bestrahlung von Tumoren Partikelstrahlen, vor allem Ionenstrahlen, insbesondere mit Protonen, α-Teilchen oder Kohlenstoffkernen, eingesetzt. Es werden Teile des Zielgebiets, die Zielpunkte, sequenziell nacheinander mit dem Strahl angefahren.

Ein Zielpunkt ist ein etwa durch Angabe von drei kartesischen Raumkoordinaten (x, y, z) definierbarer Ort, der in der Regel innerhalb des zu bestrahlenden Objekts und insbesondere innerhalb des Zielvolumens ggf. aber auch in dessen Umgebung liegt. Üblicherweise wird beim Scanning mit einem Partikelstrahl dieser durch Ablenkmagnete in zwei zueinander senkrechten Richtungen (x- und γ-Richtung) abgelenkt. Durch eine aktive Variation der Partikelenergie wird die Position des Bragg-Peaks, in dem der größte Anteil der Dosis deponiert wird, auf unterschiedliche Tiefen (z-Richtung) in dem Ziel eingestellt. Oft werden räumliche Zielgebiete schichtweise bestrahlt, wobei die die Eindringtiefe bestimmende Energie pro Schicht konstant gewählt wird (Isoenergieschicht). Bekannt ist auch das so genannte depth scanning, bei dem die hintereinander angefahrenen Zielpunkte nicht notwendig einzelnen (Isoenergie-)Schichten zugeordnet sind.

Scanning-Verfahren ermöglichen eine an die Form des Ziels angepasste Bestrahlung durch Abtasten mit einem Strahl; es werden verschiedene Scanning-Verfahren unterschieden. Bewährt hat sich insbesondere das Raster-Scanning. Bei diesem wird ein dreidimensionales Raster über das zu bestrahlende Volumen gelegt. Hier verweilt der Partikelstrahl an jedem von vielen anzufahrenden Rasterpunkten während einer vorbestimmten Zeitdauer oder deponiert an jedem dieser Rasterpunkte eine vorbestimmte Anzahl Partikel, wird aber zwischen den Rasterpunkten nicht oder nicht immer ausgeschaltet; die Offenbarung ist jedoch grundsätzlich nicht auf Raster-Scanning eingeschränkt, sondern kann auch im Zusammenhang mit Spot-Scanning, einem kontinuierlichen oder diskontinuierlichen Scanning-Verfahren oder mit einem anderen Scanning-Verfahren Anwendung finden.

Wird Raster-Scanning eingesetzt ist zu beachten, dass die Rasterpunkte sich von den Zielpunkten unterscheiden können. So fallen Zielpunkte in der Regel nur auf einen Teil der Rasterpunkte, da üblicherweise nicht sämtliche Rasterpunkte angefahren werden. Weiter können die Rasterpunkte und die Zielpunkte in unterschiedlichen Koordinatensystemen angegeben werden, etwa in einem raumfesten Koordinatensystem und einem bezogen auf das zu bestrahlende Zielvolumen. Daher müssen insbesondere Rasterpunkte und Zielpunkte nicht deckungsgleich übereinander liegen. Es ist natürlich auch möglich, sowohl Rasterpunkte als auch Zielpunkte in einem gemeinsamen Koordinatensystem anzugeben.

Ein Zielvolumen wird hier als ein Raumbereich verstanden, innerhalb dessen eine von einer Bedienperson, etwa von medizinischem Personal, festgelegte bzw. verschriebene Dosis deponiert werden soll.

Ein Partikelstrahl ist ein Strahl mit einem definierten Querschnitt aus Partikeln mit einem definierten, in der Regel schmalen Spektrum der Partikelenergie. Die Partikelenergie ist die Energie des einzelnen Partikels beim Eintritt in das zu bestrahlende Objekt.

Wenn hier auf einen Partikelstrahl Bezug genommen wird, der auf einen Zielpunkt gerichtet ist, bedeutet dies, dass der Partikelstrahl (beispielsweise durch Ablenkmagnete) in x- und y-Richtung so gelenkt wird, dass der Zielpunkt beispielsweise im Schwerpunkt oder auf einer Linie (oder deren Verlängerung) maximaler Fluenz oder Dosis liegt, und dass der Zielpunkt im Bragg-Peak des Partikelstrahls liegt; es wird auch vom Anfahren eines Zielpunktes gesprochen.

Bei der Planung einer Bestrahlung werden pro Zielpunkt bzw. pro Rasterpunkt insbesondere folgende Größen festgelegt: laterale Position (x- und y-Richtung), Energie - bestimmend die Eindringtiefe, Fokus und Teilchenzahl.

Üblicherweise soll, etwa bei der Bestrahlung eines Tumors, eine bestimmte Verteilung der Dosis, also eine Zieldosisverteilung, insbesondere eine biologisch effektive Zieldosisverteilung, über das Zielvolumen erzielt werden. Die Zieldosisverteilung wird etwa als deponierte Energie pro Volumeneinheit quantifiziert. Verbreitet ist eine Angabe einer Dosis in Joule pro Kilogramm (Gray).

Die Dosis, die als Funktion des Orts zu applizieren ist, wird von einem Anwender etwa nach einer eingehenden Untersuchung festgelegt bzw. verschrieben. Beispielsweise soll die Dosis innerhalb eines Zielvolumens insb. eines Tumors einen vorbestimmte Werteverteilung aufweisen. Außerhalb des Tumors soll die Dosis möglichst steil abfallen und vor allem in zu schützenden Organen bzw. Risikoorganen (engl.: Organ At Risk = OAR) einen vorbestimmten Maximalwert, bspw. quantifiziert als Maximaldosis, DVH-Bedingung, EUD, auch Toleranzdosis genannt, nicht überschreiten. Beispiele für Risikoorgane sind das Rektum bei Prostatabestrahlungen und bestimmte Strukturen innerhalb der Lunge bei Lungentumorbestrahlungen.

Eine Partikelstrahlvorrichtung und ein entsprechendes Planungsverfahren sind z.B. im Dokument WO 2010/049071 beschrieben.

Das Dokument "CLINICAL IMPLEMENTATION OF INTENSITY-MODULATET ARC THERAPY" beschreibt ein Verfahren zum Bereitstellen einer Intensitätsmodulierten Strahlungstherapie unter Verwendung von Rotationsstrahlen. Durch Rotieren des Strahls um den Patient herum wird mittels IMAT eine optimierte Dosisverteilung geliefert. Keile und dynamische Feldänderungen werden eingesetzt, um eine gleichförmigere Dosisverteilung im Planungszielvolumen zu erreichen.

Der Erfindung liegt die Aufgabe zu Grunde, ein vorteilhaftes Verfahren zur Planung einer Bestrahlung unter Berücksichtigung eines zu schützenden Volumens, ein entsprechendes vorteilhaftes Verfahren zur Bestrahlung, eine entsprechende vorteilhafte Vorrichtung zur Bestrahlung und ein vorteilhaftes Kontrollsystem zur Kontrolle einer solchen Vorrichtung anzugeben.

Die Aufgabe wird gelöst durch ein Verfahren zur Planung einer Bestrahlung eines Zielvolumens, wobei Intensitäten für Zielpunkte bestimmt werden, die nacheinander mit einem Strahl angefahren werden sollen, mit folgenden Schritten: Erfassen eines zu schützenden Volumens, in dem eine durch Bestrahlen des Zielvolumens erzeugte Dosis einen vorbestimmten Maximalwert nicht überschreiten soll; Bestimmen von Intensitäten für Zielpunkte so, dass innerhalb des zu schützenden Volumens die erzeugte Dosis bei einer planungsgemäßen Bestrahlung den vorbestimmten Maximalwert nicht überschreitet, wobei zum Bestimmen der Intensitäten ein Dosisbeitragsdatensatz verwendet wird, der für Zielpunkte die durch Richten des Strahls bei einer planungsgemäßen Bestrahlung auf einen der Zielpunkte mit einer vorbestimmten Intensität an anderen Orten erzeugte Dosis umfasst.

Bevorzugte Ausgestaltungen der Erfindung sind in abhängigen Ansprüchen angegeben und werden im Folgenden näher erläutert.

Ein Verfahren zur "Planung einer Bestrahlung" erfasst selbst keine Bestrahlung, sondern wird unabhängig von einer Bestrahlung durchgeführt; die Planung geht einer Bestrahlung voran. Der sprachlichen Einfachheit halber wird dies in der Beschreibung nicht immer ausdifferenziert. Die vorangehende und die folgende Beschreibung der einzelnen Verfahrensmerkmale bezieht sich sowohl auf das Planungsverfahren als auch auf das Bestrahlungsverfahren, ohne dass dies in jedem Fall explizit erwähnt ist. Die Erfindung beruht auf der Feststellung, dass teilweise die Schonung eines zu schützenden Volumens, insbesondere eines O-AR, wichtiger ist als die Abdeckung des Zielvolumens mit der geplanten Dosis. Dies kann beispielsweise der Fall sein, wenn für das Zielvolumen eine sehr hohe Dosis verschrieben wird und eventuell auch eine geringere Dosis für das Zielvolumen, insbesondere zur Tumorkontrolle, ausreichen könnte. Entsprechend kann in solchen Fällen aufgrund zwingend notwendiger Schonung von einem oder mehreren zu schützenden Volumen, insbesondere Risikoorganen, eine leichte Unterdosierung des Zielvolumens in Kauf genommen werden.

Weiter beruht die Erfindung auf der Erfahrung, dass eine Bestrahlung typischerweise in mehreren Durchgängen stattfindet, insbesondere in Fraktionen bei fraktionierter Bestrahlung, wobei in jedem Durchgang nur ein Bruchteil der gesamten zu applizierenden Dosis deponiert wird. Eine Bestrahlung in mehreren Durchgängen ist grundsätzlich vorteilhaft, insbesondere da durch statistische Effekte Fehler ausgemittelt werden können und die Normalgewebsdosistoleranz gesteigert wird.

Der zeitliche Abstand etwa zwischen Fraktionen beträgt in der Regel einen oder mehrere Tage. Eine fraktionierte Bestrahlung erstreckt sich in der Regel über mehrere Wochen. Innerhalb dieses Zeitraums kann sowohl das Zielvolumen als auch das zu schützende Volumen, etwa ein Risikoorgan, seine Position, Größe und/oder Gestalt verändern.

Um trotz Veränderungen innerhalb des Zielvolumens eine vorbestimmte Mindestdosis nicht zu unterschreiten und innerhalb eines zu schützenden Volumens eine vorbestimmte Maximaldosis nicht zu überschreiten, umfasst das Zielvolumen in der Regel einen Sicherheitssaum; das um den Sicherheitssaum erweiterte Volumen wird dann mit der gewünschten Dosis belegt. Auch das zu schützende Volumen kann um einen Sicherheitssaum ergänzt werden. Für eine Berücksichtigung möglicher Veränderungen innerhalb des langen Zeitraums, über den sich alle Durchgänge erstrecken können, sind jedoch oft sehr breite Sicherheitssäume erforderlich.

Dadurch können Situationen entstehen, die eine Bestrahlungsplanung deutlich erschweren; insbesondere können die Sicherheitssäume um die Zielvolumen mit zu schützenden Volumen nach Änderung der internen Anatomie überlappen oder aber auch Lageänderung der Zielvolumen zu einer Dosiserhöhung in dem zu schonenden Volumen führen. Weiter kann sich auch die Reichweite der Bestrahlung aufgrund anatomischer Änderungen ändern, was zu zusätzlicher Deposition in dem zu schonenden Volumen führen kann.

Je nach zu bestrahlender Struktur können entsprechende Relativbewegungen auch auf kürzeren Zeitskalen relevant sein. Wird etwa eine Person bestrahlt, so hat man Bewegungen auf der Skala von Sekundenbruchteilen (Herzschlag), von Sekunden (Atmung), und Minuten, Stunden bzw. Tagen (Änderungen anatomischer Strukturen, z.B. Darmbewegung).

Auch die Abstände zwischen den Durchgängen können recht unterschiedlich gestaltet sein, so kann etwa auch ein Durchgang pro Tag bzw. können auch mehrere Durchgänge pro Tag durchgeführt werden. Etwa bei speziellen Mehrfachbestrahlungen sind die Durchgänge sogar auf nur einige Sekunden bzw. wenige Minuten verteilt.

Auch auf diesen kürzeren Zeitskalen lässt sich die Erfindung vorteilhaft einsetzen.

Weiter beruht die vorliegende Erfindung auf der Idee, vor der Bestrahlung bzw. vor einem Durchgang der Bestrahlung, insbesondere vor einem Applizieren einer Fraktion, ein zu schützendes Volumen, in dem eine durch Bestrahlung des Zielvolumens erzeugte Dosis einen vorbestimmten Maximalwert nicht überschreiten soll, zu erfassen. Das zu schützende Volumen umfasst insbesondere ein oder mehrere zu schützende Organe bzw. Risikoorgane und optional Sicherheitssäume um diese. Für verschiedene Zielpunkte, vorzugsweise für jeden Zielpunkt, innerhalb des Zielvolumens wird die Intensität der Bestrahlung, insbesondere die Partikelzahl so bestimmt, dass innerhalb des zu schützenden Volumens die erzeugte Dosis den vorbestimmten Maximalwert nicht überschreitet. Dazu wird ein Dosisbeitragsdatensatz verwendet, der für verschiedene Zielpunkte, vorzugsweise jeden Zielpunkt, die durch Richten des Strahls auf den Zielpunkt bzw. einen der Zielpunkte mit einer vorbestimmten Partikelzahl an anderen Orten in dem Objekt bzw. Körper erzeugte Dosis umfasst.

Der Maximalwert wird auch als Schwellwert bezeichnet. Dieser kann im einfachsten Fall tatsächlich einem skalaren Dosiswert entsprechen, aber auch einer, insbesondere räumlichen, Verteilung. Der Schwellwert kann aber auch mit Blick auf die biologische Wirksamkeit, etwa unter Berücksichtigung der "equivalent uniform dose" bzw. als biologisch effektive Dosis gewählt sein. Es können auch andere eine Dosis oder Dosisverteilung entsprechende Maße verwendet werden.

Das zu schützende Volumen und/oder das Zielvolumen können insbesondere mittels Röntgentomographie, Ultraschalldiagnostik bzw. Sonographie, optischer Kohärenztomographie, Magnetresonanztomographie bzw. Kernspintomographie, Computertomographie, Positronen-Emissions-Tomographie, Single-Photon-Emission-Computered-Tomographie (SPECT), elektromagnetischer Impedanz-Tomographie (EMIT), Neutronentomographie oder anderer Verfahren erfasst werden, die zur dreidimensionalen oder auch 2D oder 4D Abbildung des zu bestrahlenden Körpers oder von Teilen desselben geeignet sind. Beim Erfassen können insbesondere Veränderungen von Position, Größe und/oder Form, natürlich auch unabhängig voneinander, zumindest entweder des zu schützenden Volumens oder des Zielvolumens gegenüber der Situation, die dem ursprünglichen Bestrahlungsplan zugrunde lag, oder gegenüber der Situation bei vorangehenden Durchgängen, erfasst werden.

Der Dosisbeitragsdatensatz kann vor dem ersten Durchgang beziehungsweise vor der ersten Fraktion erstellt und optional nach dem Erfassen des zu schützenden Volumens auf der Basis bildgebender Verfahren, korrigiert werden. Alternativ wird der Dosisbeitragsdatensatz nach dem Erfassen des zu schützenden Volumens neu erstellt.

Beim Bestimmen der Intensitäten, insbesondere der Partikelzahlen, kann neben dem Dosisbeitragsdatensatz die bei einem oder mehreren der vorangehenden Durchgänge in dem Objekt bzw. Körper erzeugte Vordosis berücksichtigt werden.

Vorzugsweise geschieht das Anfahren der Zielpunkte im Rahmen von Raster-Scanning. Jedem einzelnen Zielvolumen kann ein eigenes Raster zugeordnet sein, es ist aber auch möglich sämtliche Zielvolumen in ein Raster einzubetten.

Üblicherweise werden mehrere Rasterpunkte innerhalb eines Zielvolumens liegen, dem eine Zieldosisverteilung zugewiesen ist. Insofern kann man auch von einer Zieldosis pro Rasterpunkt, einer Rasterpunktzieldosis, sprechen. In dieser Sprechweise werden nach der Erfindung die Rasterpunktzieldosen so bestimmt, dass innerhalb des zu schützenden Volumens, der vorbestimmte Maximalwert nicht überschritten wird.

Die Zielvolumen bzw. die entsprechenden Rasterpunkte können den zu bestrahlenden Strukturen nachgeführt werden; hier wird auch von einem Anpassen gesprochen, wobei sich der Begriff Anpassen hier insb. auf die Position zu bestrahlender Punkte bezieht. Ein solches Nachführen ist etwas detaillierter unten und ausführlich in der DE 10 2006 044 139 A1 beschrieben.

Bei einem solchen Nachführen, hier beispielsweise Bestrahlung eines Tumors unter Einsatz von Raster-Scanning, werden etwa die x- und y-Positionen der Rasterpunkte sowie die erforderliche Energie für die aktuelle Anatomie festgelegt. Dabei werden nach der oben genannten DE-Schrift zunächst die Volumen zueinander registriert und die bestimmten Transformationsparameter auf die Rasterpunktpositionen in der Anatomie angewendet, wobei die erforderliche Energie anhand der aktuellen Anatomie ggf. neu zu bestimmen ist. Ggf. ist auch eine Interpolation auf ein wiederum reguläres Raster notwendig, sowie ggf. Anpassungen der Teilchenzahl pro Rasterpunkt. Dabei ist dann jedoch zu beachten, dass die vorbereiteten Dosisbeiträge nicht mittransformiert werden, sondern neu aufgesetzt beziehungsweise aus den vorbereiteten bestimmt werden. Transformationen können entweder für die Anatomie als ganzes oder aber auch für die einzelnen Zielvolumen erfolgen.
Wenn das Zielvolumen einen Sicherheitssaum umfasst, insgesamt auch Planungszielvolumen genannt, können die Intensitäten so bestimmt werden, dass die deponierte Dosis innerhalb des Planungszielvolumens, vorzugsweise in dem von dem Sicherheitssaum umgebenen inneren Bereich, einen vorbestimmten Mindestwert überschreitet und bevorzugt gleichzeitig so, dass die deponierte Dosis innerhalb des zu schützenden Volumens einen vorbestimmten Maximalwert nicht überschreitet. Wenn das zu schützende Volumen ein zu schützendes Organ und einen Sicherheitssaum um das zu schützende Organ umfasst, können die Intensitäten ferner so bestimmt werden, dass die deponierte Dosis innerhalb des zu schützenden Organs den vorbestimmten Maximalwert nicht überschreitet. Die Intensitäten werden so bestimmt, dass der Strahl auf Zielpunkte innerhalb eines Überlappbereichs des Zielvolumens und des zu schützenden Volumens bzw. Organs nicht gerichtet wird; insbesondere nicht auf einen Überlappbereich des Sicherheitssaums des Zielvolumens und des Sicherheitssaums um das zu schützende Volumen.

Wenn keine Veränderungen in dem zu bestrahlenden Objekt stattfänden, könnten die Datensätze für alle Durchgänge gleich gewählt werden. Zwischen den Durchgängen stattfindende Veränderungen bewirken Unterschiede zwischen den Datensätzen der einzelnen Durchgänge. Die beschriebene Bestimmung der Intensitäten kann als Adaptierung der Intensitäten an die Veränderungen im zu bestrahlenden Objekt bezeichnet werden. Im Laufe einer Wiederholung des beschriebenen Verfahrens vor ausgewählten oder allen Durchgängen können systematische Änderungen erkannt werden, die darauf hindeuten, dass anstelle einer wiederholten Adaptierung des Bestrahlungsplans bzw. des Datensatzes eine vollständige neue Erstellung bzw. neue Optimierung des Bestrahlungsplans vorteilhaft wäre.

Vorzugsweise wird nach jedem Durchgang die in dem Durchgang applizierte Dosis berechnet. So kann der Verlauf der Bestrahlung gut mitverfolgt werden.

Insbesondere ist es auch bevorzugt, nach jedem Durchgang der Bestrahlung die Summe aller bisher applizierten Dosen zu bestimmen, insbesondere zu berechnen.

Durch solch ein nachträgliches Bestimmen kann bei einer neuen Bestrahlungsoptimierung eine Vordosis berücksichtigt und gegebenenfalls ausgeglichen werden, indem beispielsweise die Differenz zur verschriebenen Dosis unter Berücksichtigung möglicher Fraktionierungseffekte appliziert wird.

Bei Abweichungen kann so eine neue Optimierung des Bestrahlungsplans unter Berücksichtigung der bereits applizierten Dosis durchgeführt werden. Insbesondere sind so auch gegebenenfalls vorhandene systematische Abweichungen zu erkennen, die darauf hindeuten, dass ein komplett neuer Bestrahlungsplan erstellt und optimiert werden sollte, anstatt einen vorhandenen Bestrahlungsplan von Durchgang zu Durchgang zu adaptieren beziehungsweise zu korrigieren.

Ändert sich die Lage und/oder die Form zumindest eines der Zielvolumen und/oder des zu schützenden Volumens, so kann der vorbestimmte Maximalwert für die Dosis innerhalb des zu schützenden Volumens überschritten werden, insbesondere, wenn sich die Lage zumindest eines Zielvolumens relativ zu dem zu schützenden Volumen ändert.

Besteht bereits ein Bestrahlungsplan, so ist es bevorzugt, diesen aufgrund der Lage und/oder Formänderung zu ändern, anstatt einen neuen Bestrahlungsplan zu erstellen.

Insbesondere werden dabei diejenigen Zielpunkte mit einer geänderten Dosis belegt, welche sonst zu einer überhöhten Dosis führen; ggf. ist dies nur ein kleiner Teil aller Zielpunkte. Dies betrifft vor allem die Zielpunkte eines Sicherheitssaums. Im einfachsten Fall werden Zielpunkte nicht mehr oder aber sogar neu angefahren.

Dies kann insbesondere dadurch implementiert werden, dass verschiedene räumliche Lagen und/oder Formen zumindest eines der Zielvolumen und/oder des zu schützenden Volumens von vornherein berücksichtigt werden. Insbesondere können so für Zielpunkte, die in einem der Sicherheitssäume liegen, verschiedene Dosisbeiträge, abhängig von den verschiedenen räumlichen Lagen und/oder Formen zumindest eines der Zielvolumen bzw. der zu schützenden Volumen vorberechnet werden. Dies ist vorteilhaft, da gerade bei diesen Zielpunkten oftmals eine fehlerhafte Dosis durch die Lage- bzw. Formveränderung zumindest eines der Zielvolumen und/oder des zu schützenden Volumens auftritt. Wenn dann tatsächlich eine Lageänderung bzw. Formveränderung auftritt, können die vorberechneten Dosisbeiträge zur Änderung eines bereits bestehenden Bestrahlungsplans verwendet werden. Auf diese Weise kann auf die Lage- bzw. Formveränderung schnell reagiert werden und ein Bestrahlungsplan schnell angepasst werden.

Die nach der Erfindung bestimmten Intensitäten, insbesondere Partikelzahlen, bilden vorteilhafterweise einen Datensatz, der vorzugsweise zum Steuern einer Bestrahlungsanlage zum Bestrahlen des Zielvolumens mit einem Strahl in einem kontinuierlichen oder diskontinuierlichen Prozess geeignet ist.

Der Dosisbeitragsdatensatz für die einzelnen Zielpunkte in verschiedenen Tiefen kann generisch vorliegen, so dass nicht jedes Mal eine Berechnung erfolgen muss und außerdem Dosisbeiträge aller Zielpunkte mit dem generischen Ansatz beschrieben werden können.

Der erzeugte Datensatz kann vorzugsweise als Steuerdatensatz verwendet werden, der zum Steuern einer Vorrichtung zum Bestrahlen insbesondere während des Applizierens eines Durchgangs verwendbar ist. Der Datensatz kann darüber hinaus die Koordinaten bzw. x- und y-Positionen und die Partikelenergien oder die entsprechenden z-Positionen der Zielpunkte definieren. Auch diese Koordinaten können auf der Grundlage bildge bende Verfahren, insbesondere aktueller tomographischer Daten, jeweils angepasst werden.

Es sei noch einmal betont, dass der Dosisbeitragsdatensatz insbesondere auch Dosisbeiträge außerhalb von Zielvolumen umfassen kann.

Neben der Bestrahlung von Personen oder Tieren ist auch die Bestrahlung von organischem Material überhaupt, insbesondere von Zellen, oder auch die Bestrahlung anorganischer Materialien, etwa von Kunststoffen, relevant; etwa im Rahmen der Materialforschung.

Die Aufgabe wird ebenfalls gelöst durch ein Verfahren zur Bestrahlung eines Zielvolumens, wobei Intensitäten für Zielpunkte bestimmt werden, die nacheinander mit einem Strahl angefahren werden, mit folgenden Schritten: Erfassen eines zu schützenden Volumens, in dem eine durch Bestrahlen des Zielvolumens erzeugte Dosis einen vorbestimmten Maximalwert nicht überschreiten soll; Bestimmen von Intensitäten für Zielpunkte so, dass innerhalb des zu schützenden Volumens die erzeugte Dosis den vorbestimmten Maximalwert nicht überschreitet, wobei zum Bestimmen der Intensitäten ein Dosisbeitragsdatensatz verwendet wird, der für Zielpunkte die durch Richten des Strahls auf einen der Zielpunkte mit einer vorbestimmten Intensität an anderen Orten erzeugte Dosis umfasst.

Vorzugsweise umfasst das Verfahren zur Bestrahlung das oben dargestellte Verfahren zur Planung einer Bestrahlung; insbesondere auch in einer der bevorzugten Ausgestaltungen des Planungsverfahrens. Insbesondere kann das Bestrahlungsverfahren das Erzeugen eines Datensatzes zum Steuern der Vorrichtung umfassen. Der Datensatz kann etwa auf einen Datenträger ge schrieben werden, um dann zum Steuern der Vorrichtung von diesem Gelesen zu werden. Bei einer besonderen Ausführungsform, werden die Schritte des Erzeugens und das Steuerns für jeden Durchgang, insbesondere für jede Fraktion einer fraktionierte Bestrahlung, wiederholt.

Weiter wie die Aufgabe gelöst durch eine Vorrichtung zur Bestrahlung eines Zielvolumens, mit einer Strahlenquelle und einem Kontrollsystem zur Steuerung der Vorrichtung, wobei die Vorrichtung dazu ausgelegt ist, mittels des Kontrollsystems Intensitäten für Zielpunkte zu bestimmen, die nacheinander mit einem Strahl angefahren werden, und die Vorrichtung dazu ausgelegt ist, ein zu schützendes Volumen zu erfassen, in dem eine durch Bestrahlen des Zielvolumens erzeugte Dosis einen vorbestimmten Maximalwert nicht überschreiten soll; Intensitäten für Zielpunkte so zu bestimmen, dass innerhalb des zu schützenden Volumens die erzeugte Dosis den vorbestimmten Maximalwert nicht überschreitet, wobei zum Bestimmen der Intensitäten ein Dosisbeitragsdatensatz verwendet wird, der für Zielpunkte die durch Richten des Strahls auf einen der Zielpunkte mit einer vorbestimmten Intensität an anderen Orten erzeugte Dosis umfasst.

So eine Bestrahlungsvorrichtung umfasst eine Strahlenquelle, insbesondere zur Erzeugung eines Partikelstrahls, insbesondere eines Ionenstrahls. Als Strahlenquelle kommt etwa ein Beschleuniger, insbesondere ein Synchrotron oder ein Cyclotron in Betracht.

Wird mit einem Ionenstrahl bestrahlt, so umfasst die Bestrahlungsvorrichtung auch eine Scanning-Einrichtung, kurz einen Scanner, umfassend Scanning-Magnete zum Ablenken des Ionenstrahls.

Vorzugsweise ist die Vorrichtung zur Bestrahlung zur Ausführung eines der oben dargestellten Verfahren ausgelegt; insbesondere auch in einer der bevorzugten Ausgestaltungen der Verfahren.

Ebenfalls gelöst wird die Aufgabe durch ein Kontrollsystem zur Steuerung einer Vorrichtung zur Bestrahlung eines Zielvolumens, wobei mittels des Kontrollsystem die Vorrichtung dazu ausgelegt ist, Intensitäten für Zielpunkte zu bestimmen, die nacheinander mit einem Strahl angefahren werden, ein zu schützendes Volumen zu erfassen, in dem eine durch Bestrahlen des Zielvolumens erzeugte Dosis einen vorbestimmten Maximalwert nicht überschreiten soll; Intensitäten für Zielpunkte so zu bestimmen, dass innerhalb des zu schützenden Volumens die erzeugte Dosis den vorbestimmten Maximalwert nicht überschreitet, wobei zum Bestimmen der Intensitäten ein Dosisbeitragsdatensatz verwendet wird, der für Zielpunkte die durch Richten des Strahls auf einen der Zielpunkte mit einer vorbestimmten Intensität an anderen Orten erzeugte Dosis umfasst.

Im Unterschied zu der Bestrahlungsvorrichtung umfasst das Kontrollsystem selbst keine Strahlenquelle. Wird das Kontrollsystem zur Steuerung einer Bestrahlungsvorrichtung zur Bestrahlung von Menschen oder Tieren eingesetzt, so spricht man auch von einem Therapiekontrollsystem (TCS). Vorzugsweise verfügt das Kontrollsystem über zumindest eine Einrichtung zum Feststellen der Parameter der Bestrahlungsvorrichtung, wie etwa die Scannereinstellung und Strahleigenschaften, und/oder über eine Einrichtung zum Erfassen der zu bestrahlenden Strukturen. So kann das Kontrollsystem über einen Eingang zum Empfangen eines Bildes einer bildgebenden Anlage, etwa eines Tomographen, verfügen.

Das Kontrollsystem kann etwa mit Hilfe eines Rechners oder eines Rechnersystems implementiert werden. So kann in einem solchen Rechner etwa Information betreffend die Energieaufteilung der Durchgänge und der isoenergetischen Schichten, die Zielpunkte, die Rasterpunkte, die Zieldosis pro Rasterpunkt, den Behandlungsplan und Kriterien für ein Beenden der Bestrahlung abgelegt sein. Ein Kriterium für das Beenden der Bestrahlung kann etwa das Erreichen der im Behandlungsplan festgelegten Zieldosis pro Zielpunkt sein. Es bietet sich an, die Zieldosis pro Zielpunkt in einer Tabelle abzulegen.

Vorzugsweise wird das Kontrollsystem zur Steuerung einer der oben dargestellten Vorrichtungen zur Bestrahlung ausgelegt; insbesondere auch in einer der bevorzugten Ausgestaltungen der Bestrahlungsvorrichtung. Das Kontrollsystem ist insbesondere dazu ausgelegt, ein Verfahren nach der Erfindung auf einer Vorrichtung nach der Erfindung zu steuern.

Grundsätzlich betrifft die Erfindung auch ein entsprechendes vorteilhaftes Verfahren zum Erzeugen eines Datensatzes, insbesondere auf der Basis des Planungsverfahrens, ein Verfahren zum Steuern einer Bestrahlungsvorrichtung, insbesondere auf der Basis des Bestrahlungsverfahrens, und ein entsprechendes vorteilhaftes Computerprogrammprodukt auf der Basis der Erfindung.

Grundsätzlich betrifft die Erfindung weiter auch ein Verfahren und eine Vorrichtung zum Bestimmen von Steuerparametern einer Bestrahlungsanlage für eine Bestrahlung eines vorbestimmten Zielvolumens in einem Scanning-Verfahren.

Die vorangehende und die folgende Beschreibung der einzelnen Merkmale bezieht sich sowohl auf sämtliche Gegenstände nach der Verfahrenskategorie als auch nach der Vorrichtungskategorie, ohne dass dies im Einzelnen in jedem Fall explizit erwähnt ist; die dabei offenbarten Einzelmerkmale können auch in anderen als den gezeigten Kombinationen erfindungswesentlich sein.

Die vorangehende und die folgende Beschreibung der einzelnen Merkmale bezieht sich weiter auch auf das ComputerprogrammProdukt mit Programmcode zur Durchführung bzw. Steuerung erfindungsgemäßer Verfahren und/oder zur Implementierung auf einem Rechner/Prozessor. Die Erfindung ist in den Ansprüchen definiert. Andere Ausführungsformen sind lediglich beispielhaft. Im Folgenden werden Ausführungsbeispiele mit Bezug auf die beiliegenden Figuren näher erläutert. Es zeigen:
Fig. 1 eine schematische Darstellung einer Bestrahlungsanlage;
Fig. 2 eine schematische Darstellung von Einrichtungen, die für eine Bestrahlungsplanung, zum Erzeugen einen Datensatzes oder zur Steuerung einer Bestrahlung verwendbar sind;
Fig. 3 eine schematische Darstellung von Zielpunkten, eines Zielvolumens und eines zu schützenden Volumens;
Fig. 4 eine schematische Darstellung von Zielpunkten, eines Zielvolumens und eines zu schützenden Volumens;
Fig. 5 eine schematische Darstellung von Zielpunkten, eines Zielvolumens und eines zu schützenden Volumens;
Fig. 6 eine schematische Darstellung von Zielpunkten, eines Zielvolumens und eines zu schützenden Volumens;
Fig. 7 ein schematisches Flussdiagramm eines Verfahrens zum Erzeugen eines Datensatzes und zum Steuern einer Anlage.
Fig. 8 einer Aufzählung möglicher ausgestaltender Verfahrensschritte.

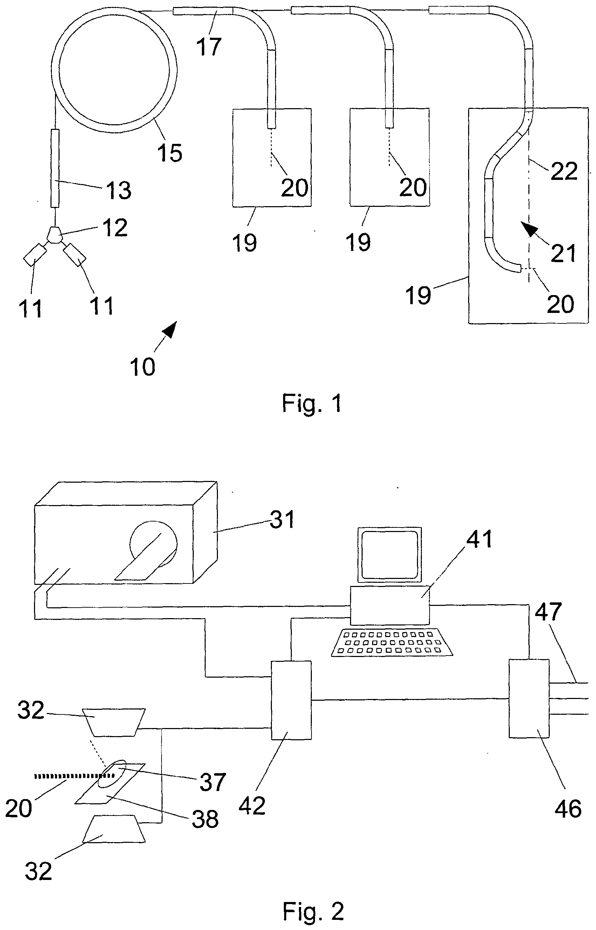

Figur 1 zeigt einen schematischen Überblick über den Aufbau einer Bestrahlungsanlage 10 als Beispiel für eine beliebige Anlage zum Bestrahlen eines Körpers, insbesondere eines tumorerkrankten Gewebes in dem Körper, mit einem Partikelstrahl. Als Partikel werden vornehmlich Ionen, beispielsweise Protonen, Pionen, Heliumionen, Kohlenstoffionen, Neonionen etc., eingesetzt.

Üblicherweise werden derartige Partikel in einer Partikelquelle 11 erzeugt. Wenn, wie in Figur 1 dargestellt, zwei Partikelquellen 11 vorhanden sind, die zwei verschiedene Ionensor ten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist.

Die von der oder einer der Ionenquellen 11 erzeugten und ggf. mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Ionen in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Ionen den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Figur 2 zeigt eine schematische Darstellung von Einrichtungen, die bei der Bestrahlungsplanung, zum Erzeugen eines Datensatzes, der Partikelzahlen und optional ferner Koordinaten von Zielpunkten in einem Zielvolumen in einem Körper definiert, und bei der Steuerung einer Bestrahlungsanlage, wie sie beispielsweise oben anhand der Figur 1 dargestellt wurde, verwendet werden können.

Mittels eines Computer-Tomographen oder Kernspin-Tomographen 31 oder mittels anderer diagnostischer Einrichtungen können Lage und Ausdehnung eines zu bestrahlenden Tumors oder eines anderen Zielvolumens ermittelt werden. Daten von dem Tomographen 31 werden unmittelbar oder nach einer Aufbereitung durch weitere, in Figur 2 nicht dargestellte Einrichtungen in einer Vorrichtung 41 zum Erstellen eines Datensatzes verarbeitet. Die Vorrichtung 41 ist beispielsweise ein Arbeitsplatz-Computer, eine Workstation oder ein anderer Computer. Optional ist die Vorrichtung 41 ferner durch ihre Benutzerschnittstelle, Software oder andere Merkmale dafür geeignet, dass medizinisches Personal dort das Zielvolumen, die zu applizierenden Dosen, die Aufteilung derselben auf mehrere Fraktionen, die Richtung der Bestrahlung und andere Details der Partikeltherapie definiert oder betrachtet bzw. bewertet, falls die Angaben vordefiniert sind; ggf. können diese dann angepasst werden.

Ein zu bestrahlender Körper 37 kann mit verschieden ausgebildeten Überwachungseinrichtungen vor, während oder nach der Bestrahlung durch die Anlage 10 überwacht werden. Beispielsweise sind eine PET-Kamera 32 (PET = Positronen-Emissions-Tomographie) und/oder ein in Figur 2 nicht dargestellter Computer-Tomograph zur Erfassung eines zu bestrahlenden Körpers 37, der auf einer Lagerfläche 38 gelagert ist, vorgesehen. Die PET-Kamera 32 und/oder ein Abstandssensor (nicht gezeigt) und die Lagerfläche 38 können innerhalb eines der oben anhand der Figur 1 dargestellten Bestrahlungsräume 19 angeordnet sein. In diesem Fall können mittels der PET-Kamera 32 und/oder Computer-Tomographen die durch einen Partikelstrahl 20 erzeugte Dosis sowie Position, Größe und Form des Zielvolumens und eines zu schützenden Volumens in dem zu bestrahlenden Körper 37 erfasst werden. Alternativ sind die PET-Kamera 32 und die Lagerfläche 38 außerhalb eines Bestrahlungsraums angeordnet. Alter nativ oder zusätzlich kann eine Tomographie des Körpers 37 mittels einer Fluoroskopie-Einrichtung, einer RöntgenEinrichtung, eines Ultraschallsensors und/oder anderer ein dreidimensionales Bild gebender Einrichtungen erstellt werden. Die Bildgebung kann direkt im Bestrahlungsraum erfolgen, jedoch auch außerhalb, bevor der Patient in den Bestrahlungsraum gebracht wird.

Der anhand der Figur 1 dargestellte Grundaufbau einer Bestrahlungsanlage 10 ist typisch für viele Partikeltherapieanlagen und andere Bestrahlungsanlagen, ein abweichender Aufbau ist aber ebenfalls möglich. Die nachfolgend beschriebenen Ausführungsbeispiele sind sowohl in Zusammenhang mit der anhand der Figur 1 dargestellten Bestrahlungsanlage und den oben anhand der Figur 2 dargestellten Einrichtungen als auch mit anderen Bestrahlungsanlagen und Einrichtungen einsetzbar.

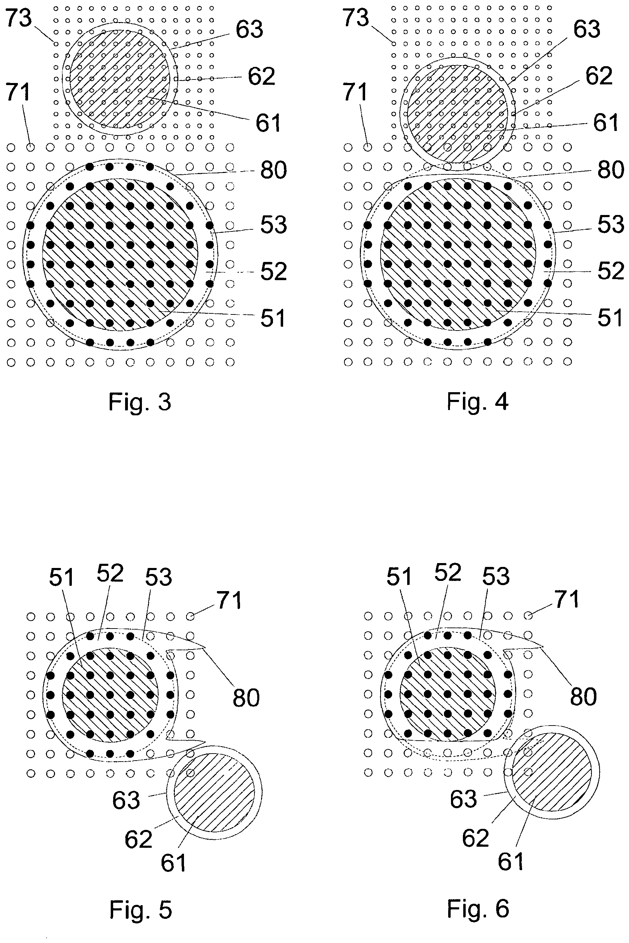

Die Figuren 3 bis 6 zeigen jeweils eine schematische Darstellung eines Aussschnitts eines Schnitts eines zu bestrahlenden Körpers. Durch kleine Kreise unterschiedlicher Durchmesser sind jeweils in einem Raster angeordnete Rasterpunkte beziehungsweise Zielpunkte 71 und Voxel 73 dargestellt. Die Zielpunkte 71 und die Voxel 73 können jeweils in kubischen, rechtwinkligen, hexagonalen oder anderen Rastern angeordnet sein. Das Raster der Zielpunkte erstreckt sich in den in den Figuren 3 bis 6 dargestellten Beispielen nur über einen Raumbereich, der ein zu bestrahlendes Gebiet, nicht jedoch den gesamten Körper umfasst. Das Raster der Voxel 73 erstreckt sich in der Regel über den gesamten Körper oder zumindest ein gesamtes Körperteil. Im Sinne einer übersichtlichen Darstellung und einer klaren Erkennbarkeit ist das Raster der Voxel jedoch nur in den Figuren 3 und 4 und dort nur außerhalb des Rasters der Zielpunkte 71 dargestellt.

Auf jeden der Zielpunkte 71 kann ein Partikelstrahl gerichtet werden. Durch die Anzahl der in einer Zeiteinheit fließenden Partikel und die Länge des Zeitintervalls, innerhalb dessen der Partikelstrahl auf den Zielpunkt 71 gerichtet ist, wird eine Partikelzahl für den Zielpunkt 71 definiert. Leere Kreise in den Figuren 3 bis 6 stellen Zielpunkte 71 dar, auf die der Partikelstrahl nicht gerichtet wird, oder die nur mit einer geringen Partikelzahl belegt werden. Ausgefüllte Kreise stellen Zielpunkte 71 dar, die mit einer hohen Partikelzahl belegt werden.

In den Figuren 3 bis 6 sind jeweils als von links oben nach rechts unten schraffierte Flächenschnitte durch einen inneren Bereich eines Zielvolumens 51 dargestellt. Die inneren Bereiche 51 sind von Sicherheitssäumen 52 umgeben. Ein innerer Bereich des Zielvolumens 51 und ein Sicherheitssaum 52 bilden zusammen jeweils ein Zielvolumen 53 bzw. auch Planungszielvolumen genannt. Zu schützende Organe 61 sind jeweils als von links unten nach recht oben schraffierte Flächen dargestellt. Jedes zu schützende Organ 61 ist von einem Sicherheitssaum 62 umgeben. Ein zu schützendes Organ 61 und ein dieses umgebender Sicherheitssaum 62 bilden jeweils zusammen ein zu schützendes Volumen 63. In den Figuren 3 bis 6 sind Isodosen 80 dargestellt, die hier einem Bereich mit einer Dosis entsprechend 95% der Maximaldosis einfassen.

Die Figuren 3 und 4 zeigen jeweils einen zur Richtung des Partikelstrahls senkrechten Schnitt bei zwei verschiedenen relativen räumlichen Anordnungen des Zielvolumens 53 und des zu schützenden Volumens 63. In Figur 3 sind das Zielvolumen 53 und das zu schützende Volumen 63 voneinander räumlich beabstandet. In Figur 4 ist der Abstand zwischen dem inneren Bereich 51 des Planungszielvolumens 53 und dem zu schützenden Organ 61 so weit verringert, dass das Zielvolumen 53 und das zu schützende Volumen 63 nicht mehr beabstandet sind, sondern teilweise überlappen.

Die Figuren 5 und 6 zeigen jeweils einen Schnitt parallel zu der Richtung eines Partikelstrahls, wobei der Partikelstrahl in den Darstellungen der Figuren 5 und 6 horizontal von links einfällt. Das Zielvolumen 53 und das zu schützende Volumen 63 sind in beiden in den Figuren 5 und 6 dargestellten Situationen voneinander räumlich beabstandet. Jedoch ist das zu schützende Volumen 63 bei der in Figur 6 dargestellten Situation gegenüber der in Figur 5 dargestellten Situation relativ zu dem Zielvolumen 63 senkrecht.zur Richtung des Partikelstrahls vertikal nach oben verschoben.

Bei den in den Figuren 5 und 6 dargestellten Schnitten seien die Dichte und die Reichweiten des Partikelstrahls inhomogen. Insbesondere seien die Reichweiten innerhalb des inneren Bereichs des Planungszielvolumens 51 höher als außerhalb desselben. Mit dem Sicherheitssaum 52 um den inneren Bereich 51 soll die Möglichkeit eines Wachstums oder einer Verlagerung des inneren Bereichs 51 berücksichtigt werden. Entsprechend werden die Partikelenergien für alle Zielpunkte 71 innerhalb des Zielvolumens 53 unter der Annahme bestimmt, dass das gesamte Zielvolumen 53 die Dichte des inneren Bereichs 51 aufweist. Wenn die Dichte innerhalb des Sicherheitssaums 52 tatsächlich nicht der Dichte des inneren Bereichs 51, sondern der geringeren Dichte der Umgebung entspricht, erhalten Bereiche weit hinter dem Zielvolumen 53 hohe Dosen. Dies ist an der Isodose 80 erkennbar. Dies würde bei der in Figur 6 dargestellten Situation zu einer unerwünschten Dosis im zu schützenden Volumen 63 führen.

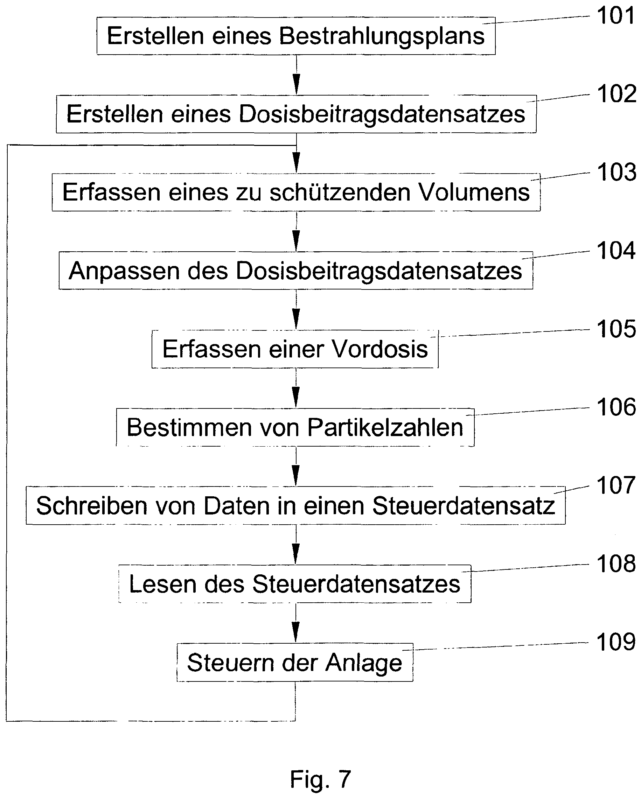

Es ist erkennbar, dass durch eine bloße Vergrößerung der Sicherheitssäume 52, 62 so, dass von vornherein die Entwicklung von der in Figur 3 bzw. Figur 5 dargestellten Situation zu der in Figur 4 bzw. Figur 6 dargestellten Situation abgedeckt ist, keine oder keine befriedigende Lösung möglich wäre. Wie nachfolgend mit Bezug auf Figur 7 näher erläutert wird, ist jedoch eine Festlegung von Partikelzahlen auf der Grundlage einer aktuellen Erfassung des zu schützenden Volumens und unter Verwendung eines Dosisbeitragsdatensatzes geeignet, um auch bei den oben anhand der Figuren 3 bis 6 dargestellten und vielen anderen Situationen gleichzeitig eine ausreichende Bestrahlung des Zielvolumens und einen ausreichenden Schutz des zu schützenden Volumens sicherzustellen. Bei den in den Figuren 4 und 6 dargestellten Situationen kann eine Folge des Verfahrens sein, dass der Partikelstrahl auf einzelne Zielpunkte 71 nicht mehr oder nur mit verminderter Partikelzahl gerichtet wird.

Figur 7 zeigt ein schematisches Flussdiagramm eines Verfahrens zum Erzeugen eines Datensatzes und zum Steuern einer Anlage. Obwohl dieses Verfahren auch bei anderen Anlagen und mit anderen Einrichtungen sowie bei anderen Situationen, als sie oben anhand der Figuren 1 bis 6 dargestellt wurden, verwendbar ist, werden nachfolgend zum einfacheren Verständnis beispielhaft Bezugszeichen aus den Figuren 1 bis 6 verwendet.

In einem ersten Schritt 101 wird ein Bestrahlungsplan für eine Bestrahlung in mehreren Durchgängen, etwa Fraktionen, erstellt. In einem zweiten Schritt 102 wird ein Dosisbeitragsdatensatz erstellt. Der Dosisbeitragsdatensatz umfasst für jeden Zielpunkt 71 und für jeden Voxel 73 die aus dem Richten des Partikelstrahls 20 mit einer vorbestimmten Anzahl von Partikeln auf den ausgewählten Zielpunkt 71 an allen Voxeln erzeugten Dosisbeiträgen. Bei n_{TP} Zielpunkten und n_{V} Voxeln ist der Dosisbeitragsdatensatz beispielsweise als Matrix mit n_{TP} Zeilen und n_{V} Spalten darstellbar.

In einem dritten Schritt 103 wird ein zu schützendes Volumen 53 erfasst, beispielsweise tomographisch. Dabei kann gleichzeitig das Zielvolumen erfasst werden.

In einem vierten Schritt 104 wird der Dosisbeitragsdatensatz an die im dritten Schritt 103 erfasste aktuelle Situation angepasst. Alternativ wird der Dosisbeitragsdatensatz neu erstellt. Im einfachsten Fall, insbesondere, wenn keine oder nur geringe Veränderungen vorliegen, kann der im zweiten Schritt 102 erstellte Dosisbeitragsdatensatz oder ein anderer Dosisbeitragsdatensatz unverändert übernommen werden.

In einem optionalen fünften Schritt wird eine in einer oder mehreren bereits applizierten Fraktionen deponierte Vordosis erfasst. Dies erfolgt beispielsweise durch eine numerische Simulation der zurückliegenden Fraktionen aufgrund der dabei jeweils tatsächlich vorliegenden Situationen und gemessenen Einstellungen der Bestrahlungsanlage. Alternativ wird die bei jeder Fraktion applizierte Dosis bereits während der Bestrahlung beispielsweise durch Positronen-Emissions-Tomographie von bei der Bestrahlung erzeugten Nukliden, die Positronen emittieren, erfasst.

In einem sechsten Schritt 106 wird für jeden Zielpunkt 71 eine Partikelzahl bestimmt. Die Partikelzahlen werden auf der Grundlage der im dritten Schritt 103 erfassten gegenwärtigen Situation, insbesondere der Positionen, Größen und Formen der zu schützenden Volumina und des Zielvolumens bestimmt. Anstelle der Partikelzahl können andere äquivalente Größen bestimmt werden, beispielsweise die elektrostatische Gesamtladung der auf den Zielpunkt gerichteten Partikel.

Optional können dabei auch zukünftige Veränderungen berücksichtigt werden. Zukünftige Veränderungen können durch eine Extrapolation bereits beobachteter Veränderungen abgeschätzt werden. Bereiche eines Zielvolumens 53, die bei späteren Fraktionen einem zu schützenden Volumen 63 voraussichtlich zu nahe kommen werden oder aus anderen Gründen nicht mehr ohne eine übermäßige Dosisbelastung eines zu schützenden Volumens bestrahlt werden können, können dann beispielsweise bereits mit der nächsten Fraktion eine erhöhte oder stark erhöhte Dosis erhalten.

Die Partikelzahlen werden unter Verwendung des im zweiten Schritt 102 erstellten und optional im vierten Schritt 104 angepassten oder neu erstellten Dosisbeitragsdatensatzes bestimmt. Beispielsweise werden die Partikelzahlen unter Verwendung des Dosisbeitragsdatensatzes iterativ so optimiert, dass möglichst innerhalb des gesamten Zielvolumens die verschriebene Dosis erreicht und möglichst innerhalb des gesamten zu schützenden Volumens eine Dosisobergrenze nicht überschritten wird. Die Optimierung kann erforderlichenfalls so erfolgen, dass innerhalb des Planungszielvolumens, oder insbesondere innerhalb eines inneren Bereichs des Planungszielvolumens, die verschriebene Dosis möglichst vollständig erreicht und innerhalb eines oder mehrerer Risikoorgane zugeordnete Dosisobergrenzen möglichst nicht überschritten werden.

Beim Bestimmen der Partikelzahlen können ferner optional die im fünften Schritt 105 erfasste Vordosis berücksichtigt werden. Die in den zukünftigen Fraktionen zu applizierende Dosis ergibt sich aus der Differenz der verschriebenen bzw. insge samt durch alle Fraktionen zu applizierenden Dosis und der in den bereits zurückliegenden Fraktionen tatsächlich (berechnet oder gemessen) applizierten Dosis. In der nächsten Fraktion, auf die sich die Partikelzahlen beziehen, ist die zu applizierende Dosis im einfachsten Fall der Quotient der in allen zukünftigen Fraktionen noch zu applizierenden Dosis und der Anzahl aller zukünftigen Fraktionen. Bei Bestrahlung aus verschiedenen Richtungen mit jeweils vorbestimmter Gesamtdosis kann diese Rechnung für jede Richtung separat durchgeführt werden.

Um das zu schützende Volumen zu schonen, sind in dem Fall nach Fig. 4 die in den Überlapp fallenden Rasterpunkt 71 inaktiv gesetzt, um den stark mit Dosis belegt im Bereich zu verkleinern, vergleiche Rückgang der Isodose. Es wird nach wie vor das Tumorvolumen jedoch nicht mehr der Sicherheitssaum des Zielvolumens mit Dosis abgedeckt.

In dem Fall nach Fig. 5 führt auch eine Inaktivierung von Rasterpunkten zu einer Verkleinerung der mit einer hohen Dosis belegten Zone, vergleiche Isodose; auch dies führt zu einer Schonung der Risikostruktur.

In einem siebten Schritt 107 werden die im sechsten Schritt 106 bestimmten Partikelzahlen in einen Datensatz geschrieben, insbesondere in einen Steuerdatensatz. Dabei können gleichzeitig Koordinaten der Zielpunkte 71 geschrieben werden.

In einem achten Schritt 108 werden die im siebten Schritt 107 in den Datensatz geschriebenen Daten gelesen. Die gelesenen Daten werden im neunten Schritt 109 zum Steuern einer Bestrahlungsanlage 10 durch eine Steuerung 46 verwendet.

Der dritte Schritt 103, der vierte Schritt 104, der fünfte Schritt 105, der sechste Schritt 106, der siebte Schritt 107, der achte Schritt 108 und der neunte Schritt 109 werden vorzugsweise vor bzw. während eines jeden Durchgangs, insb. Fraktion, wiederholt.

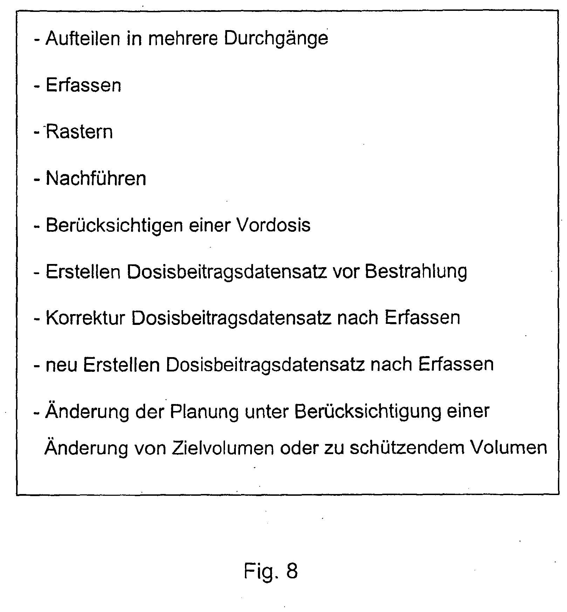

Fig. 8 zeigt eine Aufzählung ausgestaltender Verfahrensschritte darunter:
- Durchführen der Bestrahlung in mehreren Durchgängen;
- Erfassen von Veränderungen des zu schützenden Volumens und/oder des Zielvolumens;
- Raster-Scanning;
- Nachführen von Zielpunkten;
- Berücksichtigen einer Vordosis für die Bestimmung der Intensitäten, insbesondere die Partikelzahlen;
- Erstellen des Dosisbeitragsdatensatzes vor dem ersten Durchgang;
- Korrektur des Dosisbeitragsdatensatzes nach dem Erfassen;
- Änderung der Bestrahlungsplanung unter Berücksichtigung einer Änderung der Lage und/oder Form des Zielvolumens oder eines der Zielvolumen und des zu schützenden Volumens oder der zu schützenden Volumen; insbesondere bei relativer Lageänderung;
- neu Erstellen des Dosisbeitragsdatensatzes nach dem Erfassen;
- Versehen des Zielvolumens mit einem Sicherheitssaum;
- Versehen des zu schützenden Volumens mit einem Sicherheitssaum;
- Überlapp eines Sicherheitssaums eines Zielvolumens mit einem Sicherheitssaum eines zu schützenden Volumens wird nicht angefahren.

Zusammengefasst kann man insbesondere sagen, dass die Dosisbeiträge einzelner Rasterpunkte an Volumenelementen auch außerhalb der Zielvolumen bei der Bestrahlungsplanung bereits vorberechnet werden. So lässt sich anhand der aktuellen Anatomie feststellen, ob eine Risikostruktur im Vergleich zur Bestrahlungsplanung mit zusätzlicher Dosis belastet wird, wenn das aktuelle Zielvolumen bestrahlt wird. Dabei kann eine neue Berechnung der aktuellen Reichweite durchgeführt werden, insbesondere auf täglicher Basis. Rasterpunkte, welche ohne die Erfindung mit einer nicht akzeptablen Dosis in der Risikostruktur belegt werden, werden für die aktuelle Bestrahlung "nachverarbeitet", d.h. sie werden nicht oder nur mit reduzierter Intensität, insbesondere reduzierter Teilchenzahl, bestrahlt.

Insbesondere mit der dargestellten adaptiven Bestrahlung mit gescanntem Partikelstrahl können auch Bestrahlungsplanungen durchgeführt werden, in denen regelmäßig, etwa täglich, die Schonung einer Risikostruktur eine hohe Priorität hat.

Nach der Erfindung ist es möglich, die geplante Dosis für Zielvolumen zu erhöhen, auch wenn eine eventuelle Überdosierung der Risikostruktur zu erwarten wäre.

Um ohne die Erfindung eine Überdosierung einer Risikostruktur zu vermeiden müsste die Bestrahlung mit geringerer Dosis beziehungsweise kleinerem Sicherheitssäume durchgeführt werden.

Die beschriebenen Ausführungsbeispiele sind nicht nur für eine Anwendung im Rahmen einer Partikeltherapie geeignet. Sie sind darüber hinaus allgemein in Anlagen zur Bestrahlung von Materie anwendbar unabhängig davon, ob es sich um lebende oder tote, organische oder anorganische Substanz, einen Körper eines Patienten oder eines zu behandelnden Tieres, ein Phantom, eine Materialprobe oder eine Vorrichtung handelt.

### Bezugszeichenliste

- 10: Bestrahlungsanlage
- 11: Partikelquelle
- 12: Schaltmagnet
- 13: Vorbeschleuniger
- 15: Beschleuniger
- 17: Hochenergiestrahl-Transportsystem
- 19: Bestrahlungsraum
- 20: Partikelstrahl
- 21: Gantry
- 22: Achse der Gantry 21
- 31: Tomograph
- 32: PET-Kamera
- 37: Körper
- 38: Lagerfläche
- 41: Vorrichtung zum Erstellen eines Datensatzes
- 42: Einrichtung zum Bestimmen eines Bewegungsparameters
- 46: Steuerung für die Anlage 10
- 47: Steuerleitung
- 51: innerer Bereich
- 52: Sicherheitssaum
- 53: Zielvolumen/Planungszielvolumen
- 61: zu schützendes Organ/innerer Bereich
- 62: Sicherheitssaum
- 63: Zielvolumen/Planungszielvolumen
- 71: Zielpunkt
- 73: Voxel
- 80: Isodose

- 101: erster Schritt
- 102: zweiter Schritt
- 103: dritter Schritt

- 104: vierter Schritt
- 105: fünfter Schritt
- 106: sechster Schritt
- 107: siebter Schritt
- 108: achter Schritt
- 109: neunter Schritt

## Patentansprüche

1. Verfahren zur Planung einer Bestrahlung eines Zielvolumens (53) mit einem Partikelstrahl, wobei Intensitäten für Zielpunkte (70) bestimmt werden, die nacheinander mit einem Strahl (20) angefahren werden sollen, und wobei der jeweils anzufahrende Zielpunkt im Bragg-Peak des Partikelstrahls liegt, wenn der Partikelstrahl auf diesen Zielpunkt gerichtet wird, mit folgenden Schritten:
Erfassen (103) eines zu schützenden Volumens (63), in dem eine durch Bestrahlen des Zielvolumens (53) erzeugte Dosis einen vorbestimmten Maximalwert nicht überschreiten soll;
Bestimmen (106) von Intensitäten für Zielpunkte (70) so, dass innerhalb des zu schützenden Volumens (63) die erzeugte Dosis bei einer planungsgemäßen Bestrahlung den vorbestimmten Maximalwert nicht überschreitet,
wobei zum Bestimmen der Intensitäten ein Dosisbeitragsdatensatz verwendet wird, der für Zielpunkte (70) die durch Richten des Strahls (20) bei einer planungsgemäßen Bestrahlung auf einen der Zielpunkte (70) mit einer vorbestimmten Intensität an anderen Orten (73) erzeugte Dosis umfasst und der geeignet ist zum Steuern einer Bestrahlungsanlage zum Bestrahlen des Zielvolumens mit dem Partikelstrahl,
wobei bei einem Überlapp des Zielvolumens mit dem zu schützenden Volumen die Intensitäten so erzeugt werden, dass die in den Überlapp fallenden Zielpunkte inaktiv gesetzt werden, um das zu schützende Volumen zu schonen.

2. Verfahren nach Anspruch 1, bei dem die Planung eine Bestrahlung in mehreren Durchgängen, insbesondere Fraktionen, vorsieht.

3. Verfahren nach einem der vorangehenden Ansprüche, bei dem die Planung von einem Anfahren der Zielpunkte (70) mit Raster-Scanning ausgeht.

4. Verfahren nach einem der vorangehenden Ansprüche, bei dem der Dosisbeitragsdatensatz nur vor dem ersten Durchgang erstellt (102) wird.

5. Verfahren nach einem der vorangehenden Ansprüche, bei dem das Zielvolumen (53) einen inneren Bereich (51) und einen Sicherheitssaum (52) um den inneren Bereich (51) umfasst, und die Intensitäten so bestimmt (106) werden, dass bei einer planungsgemäßen Bestrahlung die innerhalb des inneren Bereichs (51) des Zielvolumens (53) deponierte Dosis einen vorbestimmten Mindestwert aufweist.

6. Verfahren nach dem vorangehenden Anspruch, bei dem das zu schützende Volumen (63) einen inneren Bereich (61) und einen Sicherheitssaum (62) um den inneren Bereich (61) umfasst, und die Intensitäten ferner so bestimmt (106) werden, dass bei einer planungsgemäßen Bestrahlung die innerhalb des inneren Bereichs (61) des zu schützenden Volumens deponierte Dosis den vorbestimmten Maximalwert nicht überschreitet.

7. Verfahren nach einem der vorangehenden Ansprüche, bei dem die Intensitäten so erzeugt werden, dass der Strahl (20) bei einer planungsgemäßen Bestrahlung auf Zielpunkte (70) innerhalb eines Überlapps eines Sicherheitssaums (52) des Zielvolumens (53) und eines Sicherheitssaums (62) des zu schützenden Volumens (63) nicht gerichtet wird.

8. Verfahren zur Bestrahlung eines Zielvolumens (53) in einem Phantom, wobei Intensitäten für Zielpunkte (70) bestimmt werden, die nacheinander mit einem Strahl (20) angefahren werden, und wobei der jeweils anzufahrende Zielpunkt im Bragg-Peak des Partikelstrahls liegt, wenn der Partikelstrahl auf diesen Zielpunkt gerichtet wird, mit folgenden Schritten:
Planung der Bestrahlung des Zielvolumens mittels des Verfahrens nach einem der Ansprüche 1 bis 7,
Schreiben des Dosisbeitragsdatensatzes auf einen Datenträger,
Lesen des Dosisbeitragsdatensatzes zum Steuern einer Vorrichtung (10) zur Bestrahlung eines Zielvolumens.

9. Vorrichtung (10) zur Bestrahlung eines Zielvolumens (53) mit einem Partikelstrahl, mit
einer Strahlenquelle (11) zur Erzeugung des Partikelstrahls und
einem Kontrollsystem (41, 42, 46,47) zur Steuerung der Vorrichtung (10),
wobei die Vorrichtung (10) dazu ausgelegt ist,
- mittels des Kontrollsystems Intensitäten für Zielpunkte (70) zu bestimmen, die nacheinander mit einem Strahl (20) angefahren werden, wobei der jeweils anzufahrende Zielpunkt im Bragg-Peak des Partikelstrahls liegt, wenn der Partikelstrahl auf diesen Zielpunkt gerichtet wird,
- ein zu schützendes Volumen (63) zu erfassen, in dem eine durch Bestrahlen des Zielvolumens (53) erzeugte Dosis einen vorbestimmten Maximalwert nicht überschreiten soll,
- Intensitäten für Zielpunkte (70) so zu bestimmen, dass innerhalb des zu schützenden Volumens (63) die erzeugte Dosis den vorbestimmten Maximalwert nicht überschreitet;
wobei zum Bestimmen der Intensitäten ein Dosisbeitragsdatensatz verwendet wird, der für Zielpunkte (70) die durch Richten des Strahls (20) auf einen der Zielpunkte (70) mit einer vorbestimmten Intensität an anderen Orten (73) erzeugte Dosis umfasst und
wobei bei einem Überlapp des Zielvolumens mit dem zu schützenden Volumen die Intensitäten so erzeugt werden, dass die in den Überlapp fallenden Zielpunkte inaktiv gesetzt werden, um das zu schützende Volumen zu schonen.

10. Kontrollsystem (41, 42, 46, 47} zur Steuerung einer Vorrichtung (10) zur Bestrahlung eines Zielvolumens (53) mit einem Partikelstrahl, wobei mittels des Kontrollsystems (41, 42, 46, 47) die Vorrichtung (10) dazu ausgelegt ist,
Intensitäten für Zielpunkte (70) zu bestimmen, die nacheinander mit einem Strahl (20) angefahren werden, wobei der jeweils anzufahrende Zielpunkt im Bragg-Peak des Partikelstrahls liegt, wenn der Partikelstrahl auf diesen Zielpunkt gerichtet wird,
ein zu schützendes Volumen (63) zu erfassen, in dem eine durch Bestrahlen des Zielvolumens (53) erzeugte Dosis einen vorbestimmten Maximalwert nicht überschreiten soll;
Intensitäten für Zielpunkte (70) so zu bestimmen, dass innerhalb des zu schützenden Volumens (63) die erzeugte Dosis den vorbestimmten Maximalwert nicht überschreitet,
wobei zum Bestimmen der Intensitäten ein Dosisbeitragsdatensatz verwendet wird, der für Zielpunkte (70) die durch Richten des Strahls (20) auf einen der Zielpunkte (70) mit einer vorbestimmten Intensität an anderen Orten (73) erzeugte Dosis umfasst und der geeignet ist zum Steuern einer Bestrahlungsanlage zum Bestrahlen des Zielvolumens mit einem Partikelstrahl,
wobei bei einem Überlapp des Zielvolumens mit dem zu schützenden Volumen die Intensitäten so erzeugt werden, dass die in den Überlapp fallenden Zielpunkte inaktiv gesetzt werden, um das zu schützende Volumen zu schonen.

## Claims

1. A method for planning an irradiation of a target volume (53) with a particle beam, wherein intensities for target points (70) are determined, which are to be targeted sequentially by a beam (20), and wherein each respective target point to be targeted is located in the Bragg peak of the particle beam when the particle beam is directed to this target point, the method comprising the steps of:
identifying (103) a volume (63) to be protected, in which a dose generated by irradiating the target volume (53) shall not exceed a predetermined maximum value;
determining (106) intensities for target points (70) in a manner so that within the volume (63) to be protected the generated dose does not exceed the predetermined maximum value during an irradiation as planned;
wherein, for determining the intensities, a dose contribution data set is used, which comprises the dose generated at other locations (73) during an irradiation as planned when directing the beam (20) to any one of the target points (70) with a predetermined intensity;
wherein if the target volume overlaps with the volume to be protected, the intensities are generated in a manner so that the target points falling into the overlap are set inactive in order to protect the volume to be protected.

2. The method according to claim 1, wherein the planning is intended for irradiation in a plurality of sessions, in particular fractions.

3. The method according to any one of the preceding claims, wherein the planning assumes that the target points (70) are targeted by raster scanning.

4. The method according to any one of the preceding claims, wherein the dose contribution data set is established (102) only prior to the first session.

5. The method according to any one of the preceding claims, wherein the target volume (53) comprises an inner zone (51) and a safety margin (52) surrounding the inner zone (51), and wherein the intensities are determined (106) in a manner so that during an irradiation as planned the dose deposited within the inner zone (51) of the target volume (53) has a predetermined minimum value.

6. The method according to any one of the preceding claims, wherein the volume (63) to be protected comprises an inner zone (61) and a safety margin (62) surrounding the inner zone (61), and wherein the intensities are furthermore determined (106) in a manner so that during an irradiation as planned the dose deposited within the inner zone (61) of the volume to be protected does not exceed the predetermined maximum value.

7. The method according to any one of the preceding claims, wherein the intensities are generated in a manner so that during an irradiation as planned the beam (20) is not directed to target points (70) within an overlap of a safety margin (52) of the target volume (53) and a safety margin (62) of the volume (63) to be protected.

8. The method for irradiating a target volume (53) in a phantom, wherein intensities for target points (70) are determined which are sequentially targeted by a beam (20), and wherein each respective target point to be targeted is located in the Bragg peak of the particle beam when the particle beam is directed to this target point, the method comprising the steps of:
planning the irradiation of the target volume using the method according to any one of claims 1 to 7;
writing the dose contribution data set to a data storage medium;
reading the dose contribution data set for controlling a device (10) for irradiating a target volume.

9. A device (10) for irradiating a target volume (53) with a particle beam, comprising:
a radiation source (11) for generating a particle beam; and
a control system (41, 42, 46, 47) for controlling the device (10);
wherein the device (10) is adapted
- to determine, by means of the control system (70), intensities for target points (70) which are sequentially targeted by a beam (20), wherein each respective target point to be targeted is located in the Bragg peak of the particle beam when the particle beam is directed to this target point;
- to identify a volume (63) to be protected, in which a dose generated by irradiating the target volume (53) shall not exceed a predetermined maximum value;
- to determine intensities for target points (70) in a manner so that within the volume to be protected (63) the generated dose does not exceed the predetermined maximum value;
wherein, for determining the intensities, a dose contribution data set is used, which comprises the dose generated at other locations (73) when directing the beam (20) to any one of the target points (70) with a predetermined intensity;
wherein if the target volume overlaps with the volume to be protected, the intensities are generated in a manner so that the target points falling into the overlap are set inactive in order to protect the volume to be protected.

10. A control system (41, 42, 46, 47) for controlling a device (10) for irradiating a target volume (53) with a particle beam, wherein by virtue of the control system (41, 42, 46, 47) the device (10) is adapted:
to determine intensities for target points (70) which are sequentially targeted by a beam (20), wherein each respective target point to be targeted is located in the Bragg peak of the particle beam when the particle beam is directed to this target point;
to identify a volume (63) to be protected, in which a dose generated by irradiating the target volume (53) shall not exceed a predetermined maximum value;
to determine intensities for target points (70) in a manner so that within the volume to be protected (63) the generated dose does not exceed the predetermined maximum value;
wherein, for determining the intensities, a dose contribution data set is used, which comprises the dose generated at other locations (73) when directing the beam (20) to any one of the target points (70) with a predetermined intensity, and which is suitable for controlling an irradiation system for irradiating the target volume with a particle beam;
wherein if the target volume overlaps with the volume to be protected, the intensities are generated in a manner so that the target points falling into the overlap are set inactive in order to protect the volume to be protected.

## Revendications

1. Procédé de planification d'une irradiation d'un volume cible (53) avec un faisceau de particules, selon lequel on détermine des intensités pour des points cibles (70) qui sont visés les uns après les autres avec un faisceau (20), et selon lequel le point cible respectif à viser se situe dans le pic de Bragg du faisceau de particules lorsque le faisceau de particules est dirigé sur ce point cible, le procédé comprenant les étapes suivantes :
détection (103) d'un volume à protéger (63), dans lequel une dose produite par irradiation du volume cible (53) ne doit pas dépasser une valeur maximale prédéfinie ;
détermination (106) d'intensités pour des points cibles (70), de manière à ce que, à l'intérieur du volume à protéger (63), la dose produite ne dépasse pas la valeur maximale prédéfinie, avec une irradiation conforme au plan,
sachant que pour la détermination des intensités, on utilise un ensemble de données de contribution de dose qui, pour des points cibles (70), comprend la dose produite à d'autres endroits (73) avec une intensité prédéfinie, en dirigeant le faisceau (20) sur l'un des points cibles (70) avec une irradiation conforme au plan, et qui est adapté pour commander une installation d'irradiation en vue de l'irradiation du volume cible avec le faisceau de particules,
sachant qu'en cas de chevauchement du volume cible avec le volume à protéger, les intensités sont produites de manière telle que les points cibles situés dans le chevauchement soient rendus inactifs pour épargner le volume à protéger.

2. Procédé selon la revendication 1, selon lequel le plan prévoit une irradiation en plusieurs passages, notamment en fractions.

3. Procédé selon l'une des revendications précédentes, selon lequel le plan se base sur un positionnement sur les points cibles (70) avec un balayage ligne par ligne.

4. Procédé selon l'une des revendications précédentes, selon lequel l'ensemble de données de contribution de dose est établi (102) uniquement avant le premier passage.

5. Procédé selon l'une des revendications précédentes, selon lequel le volume cible (53) comporte une zone intérieure (51) et une marge de sécurité (52) autour de la zone intérieure (51), et les intensités sont déterminées (106) de manière telle qu'avec une irradiation conforme au plan, la dose déposée dans la zone intérieure (51) du volume cible (53) présente une valeur minimale prédéfinie.

6. Procédé selon la revendication précédente, selon lequel le volume à protéger (63) comporte une zone intérieure (61) et une marge de sécurité (62) autour de la zone intérieure (61), et les intensités sont par ailleurs déterminées (106) de manière telle qu'avec une irradiation conforme au plan, la dose déposée dans la zone intérieure (61) du volume à protéger ne dépasse pas la valeur maximale prédéfinie.

7. Procédé selon l'une des revendications précédentes, selon lequel les intensités sont produites de manière telle qu'avec une irradiation conforme au plan, le faisceau (20) ne soit pas dirigé sur des points cibles (70) situés à l'intérieur d'un chevauchement d'une marge de sécurité (52) du volume cible (53) et d'une marge de sécurité (62) du volume à protéger (63).

8. Procédé d'irradiation d'un volume cible (53) dans un fantôme, selon lequel on détermine des intensités pour des points cibles (70) qui sont visés les uns après les autres avec un faisceau (20), et selon lequel le point cible respectif à viser se situe dans le pic de Bragg du faisceau de particules lorsque le faisceau de particules est dirigé sur ce point cible, le procédé comprenant les étapes suivantes :
planification de l'irradiation du volume cible au moyen du procédé selon l'une des revendications 1 à 7,
inscription de l'ensemble de données de contribution de dose sur un support de données,
lecture de l'ensemble de données de contribution de dose aux fins de commander un dispositif (10) destiné à l'irradiation d'un volume cible.

9. Dispositif (10) destiné à l'irradiation d'un volume cible (53) avec un faisceau de particules, comprenant
une source d'irradiation (11) destinée à produire le faisceau de particules, et
un système de contrôle (41, 42, 46, 47) destiné à commander le dispositif (10),
le dispositif (10) étant conçu pour
- déterminer, au moyen du système de contrôle, des intensités pour des points cibles (70) qui sont visés les uns après les autres avec un faisceau (20), sachant que le point cible respectif à viser se situe dans le pic de Bragg du faisceau de particules lorsque le faisceau de particules est dirigé sur ce point cible,
- détecter un volume à protéger (63), dans lequel une dose produite par irradiation du volume cible (53) ne doit pas dépasser une valeur maximale prédéfinie ;
- déterminer des intensités pour des points cibles (70), de manière à ce que, à l'intérieur du volume à protéger (63), la dose produite ne dépasse pas la valeur maximale prédéfinie,
- sachant que pour la détermination des intensités, on utilise un ensemble de données de contribution de dose qui, pour des points cibles (70), comprend la dose produite à d'autres endroits (73) avec une intensité prédéfinie, en dirigeant le faisceau (20) sur l'un des points cibles (70), et
- sachant qu'en cas de chevauchement du volume cible avec le volume à protéger, les intensités sont produites de manière telle que les points cibles situés dans le chevauchement soient rendus inactifs pour épargner le volume à protéger.

10. Système de contrôle (41, 42, 46, 47) destiné à commander un dispositif (10) d'irradiation d'un volume cible (53) avec un faisceau de particules, le dispositif (10) étant conçu, grâce au système de contrôle (41, 42, 46, 47), pour
déterminer des intensités pour des points cibles (70) qui sont visés les uns après les autres avec un faisceau (20), sachant que le point cible respectif à viser se situe dans le pic de Bragg du faisceau de particules lorsque le faisceau de particules est dirigé sur ce point cible,
détecter un volume à protéger (63), dans lequel une dose produite par irradiation du volume cible (53) ne doit pas dépasser une valeur maximale prédéfinie ;
déterminer des intensités pour des points cibles (70), de manière à ce que, à l'intérieur du volume à protéger (63), la dose produite ne dépasse pas la valeur maximale prédéfinie,
sachant que pour la détermination des intensités, on utilise un ensemble de données de contribution de dose qui, pour des points cibles (70), comprend la dose produite à d'autres endroits (73) avec une intensité prédéfinie, en dirigeant le faisceau (20) sur l'un des points cibles (70), et qui est adapté pour commander une installation d'irradiation en vue de l'irradiation du volume cible avec un faisceau de particules,
sachant qu'en cas de chevauchement du volume cible avec le volume à protéger, les intensités sont produites de manière telle que les points cibles situés dans le chevauchement soient rendus inactifs pour épargner le volume à protéger.
